(19) Europäisches Patentamt
European Patent Office
Office européen des brevets

(11) **EP 4 498 069 A1**

(12) **EUROPEAN PATENT APPLICATION**

(43) Date of publication:
**29.01.2025 Bulletin 2025/05**

(21) Application number: **24164097.8**

(22) Date of filing: **18.03.2024**

(51) International Patent Classification (IPC):
***G01N 21/47*** *(2006.01)*

(52) Cooperative Patent Classification (CPC):
**G01N 21/4788;** G01N 21/7743

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.07.2023 IT 202300015759**

(71) Applicant: **DG GROUP S.p.A.**
**28100 Novara (IT)**

(72) Inventors:
• **Radice, Dino**
**20010 Marcallo con Casone (MI) (IT)**
• **Silvestrini, Lucia**
**20017 Rho (MI) (IT)**

(74) Representative: **Praxi Intellectual Property Milano**
**Via Mario Pagano, 69/A**
**20145 Milano (IT)**

Remarks:
Amended claims in accordance with Rule 137(2) EPC.

(54) **DIFFRACTIVE SENSOR FOR SENSING TARGET ANALYTES IN A SAMPLE, AND SYSTEM AND METHOD FOR SENSING TARGET ANALYTES IN A SAMPLE BY SAID DIFFRACTIVE SENSOR**

(57)    The present invention relates to a diffractive sensor (1) for sensing a target analyte, comprising:
- a diffractive layer (3) comprising a plurality of surface regions (40) equal to each other and having a maximum dimension comprised between 5 $\mu$m and 50 $\mu$m, each surface region (40) comprising a diffractive grating (30) provided with grooves having a depth less than 200 nm, wherein in each of said surface regions (40) equal to each other the diffractive grating (30) has the same conformation;
- a receptor layer (5), overlapping the diffractive layer (3), configured to be selectively bonded to the target analyte.

The present invention also relates to a system and method for the detection of a target analyte in a sample by means of said diffractive sensor (1).

FIG. 1

Processed by Luminess, 75001 PARIS (FR)

## Description

## Technical field of the invention

The present invention relates to a sensor that exploits the optical phenomenon of diffraction by diffractive gratings for the detection of target analytes in a sample. The present invention also relates to a method and a system for sensing target analytes in a sample that exploit such a diffractive sensor.

The term "target analyte" refers to any chemical species whose presence in a sample is to be determined.

The present invention has particular application for the detection of target analytes, such as viruses or bacteria or their components, but can also be applied for the detection of target analytes of other kinds, thus not only in the medical, veterinary and diagnostic fields, but also for example in the biosafety or chemical fields, especially for the detection of traces of contaminants.

## Prior art

The detection of target analytes can be carried out according to numerous criteria and technologies, which depend on the nature of the analyte itself.

For example, reliable diagnosis of infections, performed through the detection of viral or bacterial infectious agents, is generally carried out by means of more or less complex tests, which usually require the use of sophisticated laboratory equipment and involve long times, even of the order of a few days, before results are available. Alternatively, rapid tests are also available, but these have considerably lower sensitivity than the above-mentioned tests, and thus can lead to outcomes that are not entirely reliable and are problematic from a clinical point of view.

There is therefore a particular need to make available devices that enable the detection of viruses or bacteria, as well as other target analytes, in a simple, fast, reliable and reproducible manner.

For example, EP 3907507 A1 describes a colorimetric sensor comprising a functional layer with a nanomaterial capable of generating a surface plasmon that is bioresponsive to bacteria and/or viruses, a receptor layer comprising protein substances or antibodies functioning as virus receptors, and a plasmonic nanostructured layer comprising etched nanostructures such that plasmonic colours are generated. When secretions from an individual containing the virus or bacterium are placed in contact with the functional sensor layer, the change in the latter's structure causes the change in plasmon energies and thus in the colours perceived. The change in coloration is thus indicative of the presence of the virus or bacterium in the secretion.

## Brief summary of the invention

The object of the present invention is to provide a sensor alternative to those according to the known art that enables with simplicity, rapidity and reliability the detection of generic target analytes, such as bacteria or viruses.

This and other objects are achieved by a diffractive sensor for sensing a target analyte according to claim 1, a system for sensing a target analyte according to claim 17, and a method for sensing a target analyte according to claim 22.

Dependent claims define possible advantageous embodiments of the invention.

## Brief description of the drawings

To better understand the invention and appreciate its advantages, some of its non-limiting exemplary embodiments will be described below, referring to the attached figures, in which:

Figure 1 is a schematic illustration of a diffractive sensor according to an embodiment of the invention;
Figure 2 is an enlarged view of a diffractive layer of the diffractive sensor according to an embodiment in which possible side-by-side surface regions are highlighted;
Figure 3 is an enlarged view of a diffractive layer of the diffractive sensor according to a further embodiment in which possible surface regions, partially overlapping each other, are highlighted;
Figure 4 is an enlarged view of a possible surface region of a diffractive layer of the diffractive sensor according to an embodiment;
Figure 5 is a schematic illustration of a system for sensing a target analyte in a sample according to an embodiment;
Figure 6 shows a possible diffraction image produced by the diffractive sensor according to the invention;
Figures 7 a) and b) show two possible diffraction images produced by the diffractive sensor in the absence and in presence, respectively, of a target analyte in a sample applied onto the diffractive sensor itself;
Figures 8 a)-d) are schematic illustrations, respectively, of an antibody and its fractions in which -S-S- disulfide bonds are reduced to reduced -SH disulfide bonds;
Figure 9 is a schematic illustration of the antibody fraction shown in Figure 8 b) bound to a receptor layer of the diffractive sensor according to an embodiment and of a target antigen.

## Detailed description of the invention

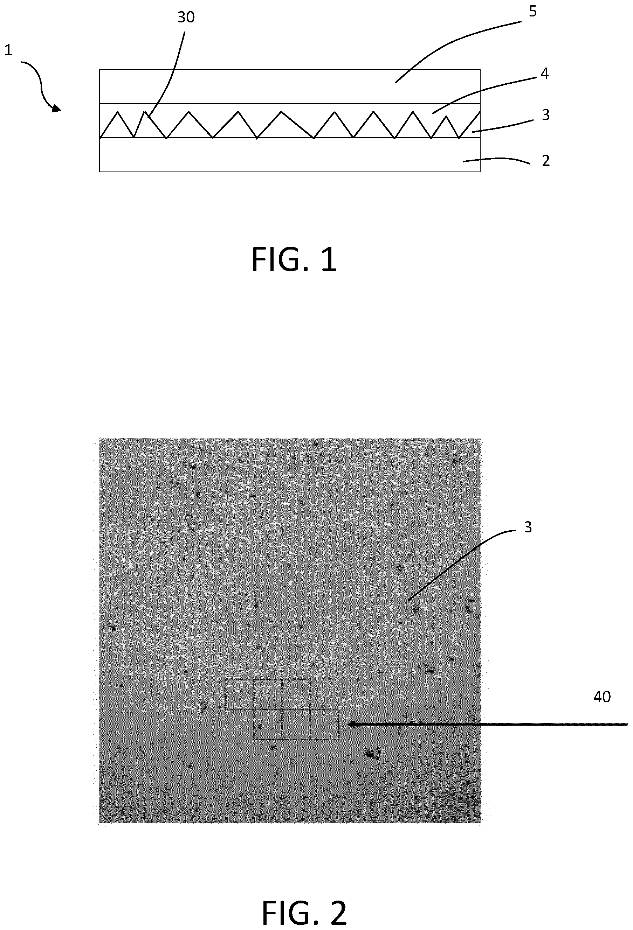

With reference to the attached Figures 1-4, a diffractive sensor for sensing a generic target analyte (or, as will be seen, a plurality of target analytes) is referred to as a whole as 1. Sensor 1 can, for example, be made in the form of a label to be affixed to a user instrument (not shown in the figures).

Sensor 1 preferably comprises a support layer 2, having the function of supporting additional overlying layers, as will be described in detail below. The support layer 2 is preferably transparent or semi-transparent and can be made of, but not limited to, polycarbonate, or PVC, or Teslin, or polyester, or the like.

Sensor 1 comprises a diffractive layer 3, preferably applied, either directly or indirectly, on the support layer 2.

Diffractive layer 3 comprises a diffractive grating 30 nanostructured, that is, provided with diffractive structures having a depth of the order of a few tens to a few hundred nanometers, as will be described in detail below.

Diffractive grating 30 can be realized:

- on a polymer film, such as a polyester film (such as polycarbonate PC, polyethylene terephthalate PET, polyvinyl chloride PVC, polypropylene PP), preferably having a thickness comprised between 5 μm and 500 μm;
- in an amorphous (e.g., a glass) or highly crystalline (e.g., a quartz) oxide material, with a thickness preferably comprised between 10 μm and 500 μm;
- in a fiberglass material.

For example, diffractive grating 30 can be made by one of the following techniques:

- ablation: this technique involves the selective removal of material and includes, for example, pulsed laser techniques such as the LIPSS (Laser-induced periodic surface structures) technique, or the LEHCEB (Low Energy High Current Electron Beam) technique;
- deposition: this technique involves the deposition of thin material on a surface and includes, for example, sputtering and optical photolithography.

Diffractive layer 3 comprises a plurality of surface regions 40 that are equal to each other, that is, in which the diffractive grating 30 has the same conformation. Furthermore, surface regions 40 have same shape and dimensions.

Surface regions 40 can have any shape, and, according to a possible embodiment, they have a square contour. Surface regions 40 preferably have a maximum dimension (identifiable as the maximum distance between two points of the contour, coinciding with a single side of the square in the case of the square contour cell) comprised between 5 μm and 50 μm, still more preferably between 30 μm and 45 μm, e.g., equal to 40 μm.

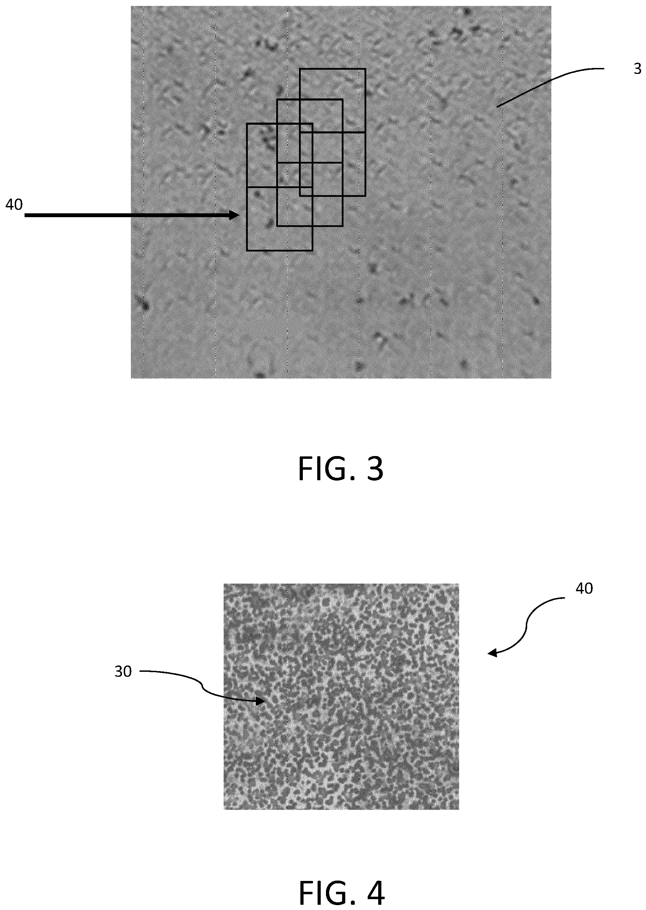

Surface regions 40 may be arranged side by side (for example, in the case of square surface regions, they may have a checkerboard arrangement, as illustrated in Figure 2), and/or they may be partially overlapping each other (in other words, a vertex of one surface region may fall within another surface region, as illustrated in Figure 3).

Referring now to a single surface region 40 (Figure 4), diffractive grating 30 has grooves that form a pattern having preferentially a random pattern, which, however, is repeated equally in each surface region 40. As visible in Figure 4, in which the darker parts represent the valleys and the lighter parts the peaks of the grooves of diffractive grating 30, no geometrically defined pattern formed by the grooves can be identified (in this sense, the pattern of the grooves of diffractive grating 30 has a "random" pattern).

The groove depth of diffractive grating 30 (i.e., the distance between the peaks and valleys) is less than 200 nm, preferably comprised between 30 nm and 200 nm, still more preferably between 100 nm and 180 nm. In an embodiment, diffractive sensor 1 further comprises a protective layer 4 to protect diffractive grating 30, preferably having a thickness comprised between 1 μm and 100 μm. The protective layer 4 can be made of a material selected from the group consisting of:

- sulfides, such as zinc sulfide (ZnS)
- oxides, such as titanium oxide (TiOz), zinc oxide (ZnO), zirconium oxide (ZrOz), and silicon oxide (SiOz);
- metals, such as gold (Au), silver (Ag), nickel (Ni), zinc (Zn), aluminum (Al), and copper (Cu).

The protective layer 4 is preferably made by deposition (e.g., under vacuum) of nanoparticles of the above-mentioned materials on the diffractive grating 30. Preferably, the nanoparticles are comprised between 4 and 30 nm in size, still more preferably smaller than or equal to 20 nm, e.g., equal to 8 nm.

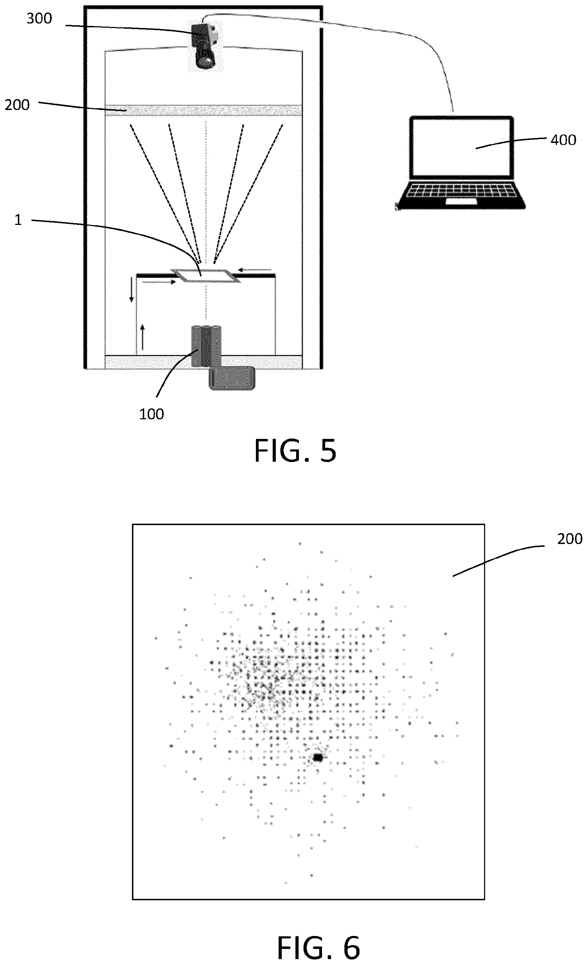

The diffractive grating 30 of the diffractive layer 3 conformed as mentioned above is such that, if a beam of monochromatic, polarized light (LASER) emitted by a laser source 100 placed posterior to the sensor 1 itself (i.e., on the side of the sensor where the support layer 2 is located) and preferably incident at 90° is passed through the sensor 1, this beam of light is diffracted into a diffraction image visible to the naked eye on a screen 200, without the need to interpose (between laser and sensor, as well as between sensor and screen) optical means, such as filters, or lenses, to amplify the image (Figure 5), according to the well-known Bragg's law:

$$n\lambda = 2d\sin\theta$$

wherein:

- n is a positive or negative integer (diffraction order);
- λ is the wavelength of the LASER beam;
- d is the distance between sensor 1 and screen 200 onto which the diffracted image is projected
- θ is the diffraction angle of order 1, which depends on the conformation of the diffractive grating.

In other words, due to the conformation of the nanometric diffractive grating, a diffractive phenomenon takes place at the macroscopic level, i.e., visible to the naked eye, similar to that which takes place, for example, at the atomic level following exposure of matter to X-rays, a phenomenon which, however, is not detectable to the naked eye.

As an example, the laser light emitted by laser source 100 can have wavelength λ equal to 532 nm (green light), however the diffraction image can be generated by irradiating sensor 1 with a laser light having any other wavelength in the visible spectrum (indicatively comprised between 390 nm and 700 nm).

An example of a diffraction image that is produced on screen 200 is shown in Figure 6. It comprises a plurality of dots whose distribution depends on the conformation of the diffractive grating 30 repeated in the surface regions 40. In the figure, a larger dot can be seen, which is the dot aligned with the laser light incident at 90° posterior to the sensor, which passes through it without being diffracted.

It is to be observed that, given the periodicity of the surface regions 40, which are repeated equally in the diffractive layer 30, the diffraction image is generated no matter where on the sensor 1 the laser light beam is aimed.

The conformation of the protective layer 4 can affect the brightness of the dots in the diffraction image on the screen 200. For example, it has been observed that employing zinc nanoparticles in the protective layer 4 results in a brighter diffraction image than if gold nanoparticles are used, but the distribution of dots in the diffraction image remains the same. Therefore, the conformation of the diffractive gratings in the surface regions 40 causes sensor 1 to produce a kind of unique fingerprint of sensor 1, that is, precisely the diffraction image. Changing the random pattern conformation of the diffractive grating 30 will also result in a different distribution of dots in the diffraction image.

It is to be observed that diffractive layer 3 is conformed so that the light diffracted by the sensor is polarized.

It should also be noted that the diffraction image can be obtained not only by crossing the sensor by the laser beam from the back side, as described above, but also by reflection of the same, i.e., by pointing a laser beam at an angle to the front surface of the sensor (i.e., on the side opposite to the bottom side, for example, on the side where the protective layer 4 may be arranged). In both cases, a diffraction image having the characteristics said above is obtained.

Returning now to Figure 1, diffractive sensor 1 further comprises a receptor layer 5 overlapping and directly in contact with diffractive layer 3, or overlapping and directly in contact with protective layer 4, where present.

Receptor layer 5 is able to bind selectively to the target analyte to be detected and not to substances of a different nature. The target analyte can be contained in a sample, such as a biological solution (e.g., a saliva or blood or urine sample), which can be deposited, for example smeared or rubbed, on receptor layer 5.

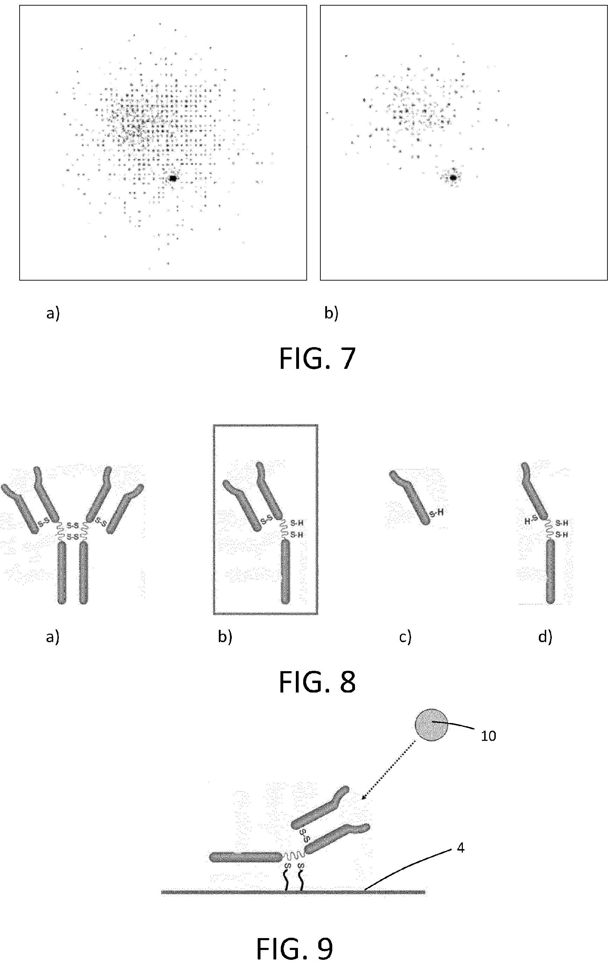

In this way, if the target analyte is not present in the sample, receptor layer 5 is not altered and diffractive sensor 1, when subjected to a laser light beam as described above, produces a first diffraction image on the screen, visible to the naked eye. On the other hand, when the target analyte is present in the sample, it binds to receptor layer 5, and as a result diffractive sensor 1 produces a second diffraction image different from the first, again visible to the naked eye, and comparable with the diffraction image of the first condition. Figure 7 shows a comparison between a diffraction image produced in the case of absent target analyte (Figure 7a)) and in the case of target analyte present and bound to receptor layer 5 (Figure 7b)). As can be seen, the number and/or distribution and/or light intensity of the dots visible in the diffraction image of the first and second cases are different. Therefore, from the simple visual comparison, which can also be made with the naked eye, of the two diffraction images, it is possible to determine whether the target analyte is present in the sample and has bound to receptor layer 5 of diffractive sensor 1, or not.

In general, the diffraction image produced by diffractive layer 3 alone is different from the diffraction image produced as a result of the application of receptor layer 5 and is also different from the diffraction image produced in the case where receptor layer 5 binds to the target analyte. Therefore, the sensor comprising diffractive layer 3, with or without protective layer 4, and lacking receptor layer 5, is as such capable of producing a diffraction image which is peculiar to the nanometer diffractive grating, which constitutes a kind of fingerprint of the sensor.

It should be observed that the diffraction images produced in the presence and absence of the target analyte may also differ from each other in coloration. However, this is a possible secondary effect to the variation in the diffraction image. Preferably, the diffraction image, whose colour depends on the laser light used (e.g., for a wavelength λ = 532 nm, the colour is green), is converted to a gray scale.

It should also be noted that, in order to prevent any pollutants other than the target analyte, which are present in the sample and/or in the environment where the sensor is located, from altering the diffraction image, it is preferable that receptor layer 5, following the application of the sample where the possible target analyte presence is to be detected, is washed in an appropriate manner, some possible examples of which will be given below. In this manner, substances or molecules or contaminants, which cannot bind to receptor layer 5 (which is able to selectively bind only to the target analyte), are removed.

The nature of receptor layer 5, in particular its chemical composition, as well as the manner of washing (where applicable), may differ depending on the target analyte being sought. In general, receptor layer 5 may include antibodies, proteins, molecules with key-lock action, chelating compounds or other chemical or biological substances that have chelating functions.

By way of non-limiting example, target analytes may include:

- human chorionic gonadotropin (hCG): in this case receptor layer 5 may include a monoclonal antibody specific for hCG, and sensor 1 will be suitable for pregnancy testing;
- Tetanus toxoid: in this case receptor layer 5 may include a monoclonal antibody specific for tetanus toxin, and sensor 1 will be able to detect tetanus toxin;
- nucleocapsid (N) protein of SARS-CoV-2: in this case, receptor layer 5 may include a monoclonal antibody specific to the nucleocapsid protein of SARS-CoV-2, and sensor 1 will be able to detect the presence of SARS-CoV-2 virus in a biological sample.

Therefore, the inclusion in receptor layer 5 of the above-mentioned antibodies enables the latter to selectively detect the presence of these target analytes in the sample that is applied onto receptor layer 5. In general, a suitable antibody against a component to be detected by the sensor can also be specifically produced.

In general, in the case where the target analyte is a specific antigen, the receptor layer may include the antibody specific for that antigen and nonspecific for antigens other than the target antigen, which is intended to be detected by the sensor 1. It is therefore necessary for the antibody to be firmly bound to the diffractive layer 3 for the detection to be reliable. Advantageously, such firm binding can be achieved by binding the antibody to the nanoparticles, preferably metallic, of the protective layer 4.

Antibodies are modular-structured protein complexes with a common basic structure, but showing variability in specific regions capable of binding to specific antigens. In general, antibodies exhibit a Y conformation, with a central stem and two side branches. They comprise four chains covalently linked by -S-S- disulfide bridges and are unable to bind to a metal, such as gold, unless processed in advance.

One possible pretreatment of the antibody for the purpose of its binding to the protective layer 4, in particular to the metal nanoparticles thereof, involves breaking the -S-S- disulfide bond of a portion of the antibody by reducing it to a reduced -SH disulfide bond, which is instead capable of binding to the metal, in particular forming very strong thiol bonds with the latter. If the -S-S- disulfide bonds of the antibody are broken, four different portions of the antibody are obtained (Figures 8a)-d)), in which only half of the whole antibody (shown in Figure 8b)), i.e., the portion of the antibody that includes a single side branch i.e., a Fab' fragment, and half of the stem, i.e., half of the Fc fragment, is suitable to form a stable bond with the metal of the protective layer 4 on the one hand and to maintain the ability to bind to the target antigen 10 on the other (Figure 9). Disulfide -S-S- bonds are then reduced in a hinge region that holds together the two heavy chains of the antibody, which are separated.

Since all antibody portions obtainable by breaking disulfide-S-S- bonds differ in size, it is possible to select the desired antibody portion, for example, by filtering precisely according to size.

The reduction of -S-S- disulfide bonds to reduced -SH disulfide bonds can be accomplished by molecular techniques known per se (such as in particular: use of reducing agents, polyacrylamide gels, ultrafiltration devices). In this regard, see, for example, the following papers, the contents of which are fully incorporated herein by reference:

- Rafael Antonio Salinas Dominguez, Miguel Ángel Dominguez Jiménez, Abdú Orduña Diaz: "Antibody Immobilization in Zinc Oxide Thin Films as an Easy-Handle Strategy for Escherichia coli Detection" - ACS Omega 2020, 5, 20473-20480;
- Anke K. Trilling, Jules Beekwildera, Han Zuilhof: "Antibody orientation on biosensor surfaces: a minireview" - Analyst, 2013, 138, 1619;
- Lu Zhang, Yacine Mazouzi, Mich&le Salmain, Bo Liedberg, Souhir Boujday: "Antibody-Gold Nanoparticle Bioconjugates for Biosensors: Synthesis, Characterization and Selected Applications" - Biosensors and Bioelectronics 165 (2020) 112370;
- Asta Makaraviciute, Carolyn D. Jackson, Paul A. Millner, Almira Ramanaviciene: "Considerations in producing preferentially reduced half-antibody fragments" - Journal of Immunological Methods 429 (2016) 50-56;
- Monalisa Pal, Sanghee Lee, Donghoon Kwon, Jeongin Hwang, Hyeonjeong Lee, Seokyung Hwang, Sangmin Jeon: "Direct immobilization of antibodies on Zn-doped Fe3O4 nanoclusters for detection of pathogenic bacteria" - Analytica Chimica Acta 952 (2017) 81-87;
- Md. Morsaline Billah, Christopher S. Hodges, Henry C.W. Hays, P.A. Millner: "Directed immobilization of reduced antibody fragments onto a novel SAM on gold for myoglobin impedance immunosensing."- Bioelectrochemistry 80 (2010) 49-54;
- Hongcheng Liu & Kimberly May (2012): 'Disulfide bond structures of IgG molecules' - mAbs, 4:1, 17-23;
- Roberto Reverberi, Lorenzo Reverberi: "Factors affecting the antigen-antibody reaction" - Blood Transfus 2007; 5: 227-240;
- Harsh Sharma, Raj Mutharasan: "Half Antibody Fragments Improve Biosensor Sensitivity without Loss of Selectivity" - Anal. Chem. 2013, 85, 2472-2477;

- Woochang Lee, Byung-Keun Oh, Won Hong Lee, Jeong-Woo Choi: "Immobilization of antibody fragment for immunosensor application based on surface plasmon resonance."- Colloids and Surfaces B: Biointerfaces 40 (2005) 143-148;
- Yuanli Song, Hui Cai, Zhijun Tan, Nesredin Mussa, Zheng-Jian Li: "Mechanistic insights into inter-chain disulfide bond reduction of IgG1 and IgG4 antibodies" - Applied Microbiology and Biotechnology (2022) 106:1057-1066;
- Nicholas G. Welch, Judith A. Scoble, Benjamin W. Muir, et al: "Orientation and characterization of immobilized antibodies for improved immunoassays" - Biointerphases 12, 02D301 (2017);
- Arkady A. Karyakin, Galina V. Presnova, Maya Yu. Rubtsova, Alexey M. Egorov: "Oriented Immobilization of Antibodies onto the Gold Surfaces via Their Native Thiol Groups" - Anal. Chem. 2000, 72, 3805-3811;
- Nur Mustafaoglu, Tanyel Kiziltepe, Basar Bilgicer: "Site-Specific Conjugation of Antibody on Gold Nanoparticle Surface for One-Step Diagnosis of Prostate Specific Antigen with Dynamic Light Scattering" - Nanoscale. 2017 June 29; 9(25): 8684-8694;
- Harry W Schroeder Jr, Lisa Cavacini: "Structure and Function of Immunoglobulins" - Allergy Clin Immunol. 2010 February; 125(2 0 2): S41-S52.

In cases where receptor layer 5 comprises antibodies or other proteins, the previously mentioned washing can, for example, be done by the use of PBS (Phosphate Buffered Saline), by immersion and possibly by subsequent centrifugation of the sensor.

It should be noted that, according to one possible embodiment, receptor layer 5 is able to selectively bind to a plurality of different target analytes, for example in different areas of receptor layer 5, and is configured such that that, depending on the bound target analyte, the diffraction image produced is different. For example, receptor layer 5 may include different antibodies positioned in such a way that receptor layer 5 is able to selectively bind to different target antigens.

Although the diffraction images produced by the sensor are visible to the naked eye, they can be usefully detected by an automated or partially automated system. As an example, a possible system for sensing a target analyte in a sample is shown in Figure 5, which comprises laser source 100, screen 200 (preferably satin), and a vision system capable of acquiring the diffraction image produced by sensor 1, e.g., a video camera 300 pointed at screen 200, connected to a control unit of, for example, a computer 400. The latter control unit can possibly perform grayscale conversion of the diffraction image. Such a system may be portable, e.g., to perform onsite analysis. In such a case, power supplies and/or batteries may be provided for the above-mentioned equipment.

According to a further aspect of the present invention, a method for sensing a target analyte in a sample comprises the steps of:

- providing a diffractive sensor 1 in accordance with the embodiments described above;
- applying the sample to the receptor layer 5 of the diffractive sensor 1;
- hitting the diffractive sensor 1 with a beam of laser light - for example, emitted by source 100 - so that diffractive sensor 1 produces a diffraction image visible to the naked eye - for example, on screen 200;
- comparing the diffraction image produced by the diffractive sensor 1 with a reference diffraction image obtained by hitting the diffractive sensor with an equal beam of laser light in the absence of the target analyte on the receptor layer 5. This comparison can be made by a naked-eye operator or, preferably, by an automated control unit, e.g., of the computer 400;
- determining the presence of the target analyte in the sample if the diffraction image produced by diffractive sensor 1 is different from the reference diffraction image.

In an embodiment, the method comprises a step of converting diffraction images to a grey scale, to remove the coloration that depends on the wavelength of the laser light beam. This step can be carried out, for example, by the control unit.

In the case where the receptor layer is able to selectively bind to a plurality of target analytes, the method may further comprise the additional steps of comparing the diffraction image produced by the sensor with a plurality of stored diffraction images (each corresponding to a specific target analyte), and determining the presence of a specific target analyte from the plurality of target analytes detectable by the sensor if the diffraction image produced by the sensor matches the stored diffraction image for that specific target analyte.

In an embodiment, following the step of applying the sample to receptor layer 5 and prior to the step of hitting diffractive sensor 1 with a laser light beam, the method comprises an additional step of washing diffractive sensor 1 to remove substances or molecules or contaminants other than the target analyte from receptor layer 5 that might interfere with the final diffraction image, even if only as background noise. Such a washing step can, for example, be carried out by using buffer solutions (e.g., PBS), ionic or non-ionic detergents, or by mild surfactants, according to appropriate procedures and timing.

The diffractive sensor, system and method according to the present invention can find application in a variety of fields, of which some non-limiting examples are given below:

- Diagnostics in medical or veterinary settings:

    ∘ diagnosis, by demonstration of the pathogen or its components, of bacterial (such as: Lyme disease, Brucellosis, Syphilis), viral (such as: HIV, Hepatitis A,B,C) and fungal (such as pathogenic yeast infections) infections;

○ detection of viruses (such as: Coronavirus, HIV, Hepatitis, Ebola, Norovirus, Influenza, West Nile, Zika);

○ diagnosis, by demonstration of specific clinically validated markers, of autoimmune diseases, due to abnormal production by the immune system of autoantibodies that destroy cells in the body (for example: autoantibodies that destroy insulin-producing cells in the pancreas in type I diabetes);

○ detection and quantification of hormones (e.g., human chorionic gonadotropin (hCG), follicle-stimulating hormone (FSH), testosterone);

○ screening of donated blood (e.g., for detection of viral infections, such as HIV);

○ detection of drugs (for example: amphetamines, cocaine);

○ detection of tumoral markers (for example: prostate specific antigen (PSA) for prostate cancer diagnosis);

○ diagnostics in the veterinary field, for example: detection of African swine fever, avian fever, bovine parvovirus, canine adenovirus, coronavirus, equine infectious anemia, feline leukemia, etc.).

- DNA search;
- Non-diagnostic applications:

   ○ Biosafety:

      ■ Surveillance of epidemics;
      ■ Biological weapons/bioterrorism testing;
      ■ Testing in cosmetics (for example: testing for metal contamination);

   ○ Detection of contaminants:

      ■ Detection and screening of contaminants in food (eg: *Salmonella sp., Escherichia coli, Campylobacter sp., Staphylococcus aureus*);
      ■ Detection and screening of environmental pollutants (e.g., detection of heavy metals in water or soil);
      ■ Pesticide detection;
      ■ Gunpowder detection (for example: nitrate detection by specific enzymes, such as nitrate reductase).

It should be noted that in this description and the attached claims the term "overlapping," referring to the layers of sensor 1, is not intended to imply necessarily also a direct contact between the mentioned overlapping layers. Such layers may therefore be in direct contact with each other, or, alternatively, they may have one or more intermediate layers arranged between them, without prejudice to their overlapping.

It should also be noted that, for the purpose of this description and the appended claims, except where otherwise indicated, all numbers expressing quantities, measures, percentages, and so on, are to be considered as modified in all cases by the term "about." Further, all ranges include any combination of the maximum and minimum points disclosed and include any intermediate ranges within them, which may or may not be specifically enumerated.

This disclosure, in accordance with at least one of the above aspects, may be implemented in accordance with one or more of the described embodiments, optionally combined with each other.

For the purpose of this description and the attached claims, the terms "an" or "one" should be read as including one or at least one and the singular includes the plural, unless it is obvious that it is intended otherwise.

To the description given above of the diffractive sensor and the system and method for sensing a target analyte the skilled person, in order to meet specific contingent needs, may make numerous additions, modifications, or substitutions of elements with functionally equivalent ones, without, however, departing from the scope of the attached claims.

**Claims**

1.  Diffractive sensor (1) for sensing a target analyte, comprising:

    - a diffractive layer (3) comprising a plurality of surface regions (40) equal to each other and having a maximum dimension comprised between 5 μm and 50 μm, each surface region (40) comprising a diffractive grating (30) provided with grooves having a depth less than 200 nm, wherein in each of said surface regions (40) equal to each other the diffractive grating (30) has the same conformation;
    - a receptor layer (5), overlapping the diffractive layer (3), configured to be selectively bonded to the target analyte.

2.  Diffractive sensor (1) according to the preceding claim, wherein the grooves of the diffractive grating (30) of each surface region (40) form a pattern having a random trend.

3.  Diffractive sensor (1) according to claim 1 or 2, wherein each of said surface regions (40) has a maximum dimension comprised between 30 μm and 45 μm.

4. Diffractive sensor (1) according to anyone of the preceding claims, wherein said surface regions (40) have a square shape and said maximum dimension is a side of the square.

5. Diffractive sensor (1) according to anyone of the preceding claims, wherein said surface regions (40) are placed side by side.

6. Diffractive sensor (1) according to anyone of the preceding claims, wherein said surface regions (40) are partially overlapped.

7. Diffractive sensor (1) according to anyone of the preceding claims, wherein the grooves of the diffractive grating (30) of each surface region (40) of the diffractive layer (3) have a depth comprised between 30 nm and 200 nm, preferably comprised between 100 nm and 180 nm.

8. Diffractive sensor (1) according to anyone of the preceding claims, wherein said diffractive layer (3) comprises:

   - a polymer film having a thickness comprised between 5 $\mu$m and 500 $\mu$m selected among the group consisting of: polycarbonate, polyethylene terephthalate, polyvinylchloride, polypropylene; or
   - an amorphous or crystalline material having a thickness comprised between 10 $\mu$m and 500 $\mu$m; or
   - a fiberglass material.

9. Diffractive sensor (1) according to anyone of the preceding claims, further comprising a protective layer (4) for protecting the diffractive grating (30) overlapping and in direct contact with the diffractive layer (3).

10. Diffractive sensor (1) according to the preceding claim, wherein said protective layer (4) is made of:

    - a sulphur; or
    - an oxide selected among the group consisting of: titanium oxide, zinc oxide, zirconium oxide, silicon oxide; or
    - a metal, selected in the group consisting of: gold, silver, nickel, zinc, aluminum, copper.

11. Diffractive sensor (1) according to claim 9 or 10, wherein said protective layer (4) comprises nanoparticles deposited on the diffractive grating (30) of the diffractive layer (3).

12. Diffractive sensor (1) according to the preceding claim, wherein said nanoparticles of the protective layer (4) have dimensions comprised between 4 and 30, preferably less than 20 nm.

13. Diffractive sensor (1) according to anyone of claims from 9 to 12, wherein said receptor layer (5) overlaps and is in direct contact with the protective layer (4).

14. Diffractive sensor (1) according to anyone of the preceding claims, wherein said target analyte is a specific antigen and the receptor layer (5) comprises an antibody adapted to be bonded to said specific antigen.

15. Diffractive sensor (1) according to the preceding claim, wherein said antibody comprises a fraction Fab' and half of the fraction Fc of the antibody in which the disulfide bond -S-S- is reduced to a reduced disulfide bond -SH adapted to be bonded to the protective layer (4).

16. Diffractive sensor (1) according to anyone of the preceding claims, further comprising a transparent or semi-transparent support layer (2), wherein said diffractive layer (3) overlaps said support layer (2).

17. System for detecting a target analyte, comprising:

    - a diffractive sensor (1) according to anyone of the preceding claims;
    - a source (100) of a laser light beam having a wavelength comprised in the visible spectrum, arranged so that the diffractive sensor (1) is hit by the laser light beam and generates a diffraction image;
    - a screen (200) placed at a distance from the diffractive sensor (1) and arranged so that the diffractive image generated by the same is projected on it.

18. System according to claim 17, wherein said laser light source (100) is arranged behind the diffractive sensor (1), so that the laser light beam hits the sensor at 90° and the diffraction image is generated by the laser light beam crossing the diffractive sensor (1).

19. System according to claim 17, wherein said laser light source (100) is arranged in front of the sensor (1), so that the laser light beam diagonally hits the diffractive sensor (1), and the diffractive image is generated by reflection from the diffractive sensor (1).

20. System according to anyone of claims from 17 to 19, further comprising a vision system (300) capable of detecting the diffraction image projected on the screen (200).

21. System according to the preceding claim, further comprising a control unit (400) operatively connected to the vision system (300) and configured to compare the diffraction image projected on the screen (200) to a reference diffraction image, and to determine the presence of the target analyte on the receptor layer (5) of the diffractive sensor (1) if the detected diffraction image is different from the reference diffraction image.

22. Method of detecting a target analyte in a sample, comprising the steps of:

    - providing a diffractive sensor (1) according to anyone of claims from 1 to 17;
    - applying the sample to the receptor layer (5) of said diffractive sensor (1);
    - hitting the diffractive sensor (1) with a laser light beam having a wavelength comprised in the visible spectrum, so that the diffractive sensor (1) generates a diffraction image visible to a naked eye;
    - comparing the diffraction image generated by the diffractive sensor (1) to a reference diffraction image obtained by hitting the diffractive sensor (1) with an equal laser light beam without the target analyte on the receptor layer (5);
    - determining the presence of the target analyte in the sample if the diffraction image generated by the diffractive sensor (1) is different from the reference diffraction image.

23. Method according to the preceding claim, comprising, after the step of applying the sample on the receptor layer (5) and before the step of hitting the diffractive sensor (1) with a laser light beam, a step of washing the diffractive sensor (1) for removing polluting substances or molecules or agents different from the target analyte from the receptor layer (5).

24. Method according to claim 22 or 23, wherein the receptor layer (5) of the diffractive sensor (1) is capable to be selectively bonded to a plurality of target analytes, the method further comprising the steps of:

    - comparing the diffraction image produced by the diffractive sensor (1) to a plurality of stored diffraction images, each corresponding to a specific target analyte of the plurality of target analytes;
    - determining the presence of a specific target analyte of the plurality of target analytes if the diffraction image generated by the diffractive sensor coincides with the stored diffraction image for that specific target analyte.

**Amended claims in accordance with Rule 137(2) EPC.**

1. Diffractive sensor (1) for sensing a target analyte, comprising:

    - a diffractive layer (3) comprising a plurality of surface regions (40) equal to each other, each surface region (40) comprising a diffractive grating (30) provided with grooves, wherein in each of said surface regions (40) equal to each other the diffractive grating (30) has the same conformation;
    - a receptor layer (5), overlapping the diffractive layer (3), configured to be selectively bonded to the target analyte,
    **characterized in that** each surface region (40) has a maximum dimension comprised between 30 $\mu$m and 45 $\mu$m and **in that** the grooves of the diffractive grating (30) of each surface region (40) have a depth comprised between 100 nm e 180 nm and form a pattern having a random trend.

2. Diffractive sensor (1) according to claim 1, wherein said surface regions (40) have a square shape and said maximum dimension is a side of the square.

3. Diffractive sensor (1) according to anyone of the preceding claims, wherein said surface regions (40) are placed side by side.

4. Diffractive sensor (1) according to anyone of the preceding claims, wherein said surface regions (40) are partially overlapped.

5. Diffractive sensor (1) according to anyone of the preceding claims, wherein said diffractive layer (3) comprises:

    - a polymer film having a thickness comprised between 5 $\mu$m and 500 $\mu$m selected among the group consisting of: polycarbonate, polyethylene terephthalate, polyvinylchloride, polypropylene; or
    - an amorphous or crystalline material having a thickness comprised between 10 $\mu$m and 500 $\mu$m; or
    - a fiberglass material.

6. Diffractive sensor (1) according to anyone of the preceding claims, further comprising a protective layer (4) for protecting the diffractive grating (30) overlapping and in direct contact with the diffractive layer (3).

7. Diffractive sensor (1) according to the preceding claim, wherein said protective layer (4) is made of:

- a sulfide; or
- an oxide selected among the group consisting of: titanium oxide, zinc oxide, zirconium oxide, silicon oxide; or
- a metal, selected in the group consisting of: gold, silver, nickel, zinc, aluminum, copper.

8. Diffractive sensor (1) according to claim 6 or 7, wherein said protective layer (4) comprises nanoparticles deposited on the diffractive grating (30) of the diffractive layer (3).

9. Diffractive sensor (1) according to the preceding claim, wherein said nanoparticles of the protective layer (4) have dimensions comprised between 4 and 30, preferably less than 20 nm.

10. Diffractive sensor (1) according to anyone of claims from 6 to 9, wherein said receptor layer (5) overlaps and is in direct contact with the protective layer (4).

11. Diffractive sensor (1) according to anyone of the preceding claims, wherein said target analyte is a specific antigen and the receptor layer (5) comprises an antibody adapted to be bonded to said specific antigen.

12. Diffractive sensor (1) according to the preceding claim, wherein said antibody comprises a fraction Fab' and half of the fraction Fc of the antibody in which the disulfide bond -S-S- is reduced to a reduced disulfide bond -SH adapted to be bonded to the protective layer (4).

13. Diffractive sensor (1) according to anyone of the preceding claims, further comprising a transparent or semi-transparent support layer (2), wherein said diffractive layer (3) overlaps said support layer (2).

14. System for detecting a target analyte, comprising:

    - a diffractive sensor (1) according to anyone of the preceding claims;
    - a source (100) of a laser light beam having a wavelength comprised in the visible spectrum, arranged so that the diffractive sensor (1) is hit by the laser light beam and generates a diffraction image;
    - a screen (200) placed at a distance from the diffractive sensor (1) and arranged so that the diffractive image generated by the same is projected on it.

15. System according to claim 14, wherein said laser light source (100) is arranged behind the diffractive sensor (1), so that the laser light beam hits the sensor at 90° and the diffraction image is generated by the laser light beam crossing the diffractive sensor (1).

16. System according to claim 14, wherein said laser light source (100) is arranged in front of the sensor (1), so that the laser light beam diagonally hits the diffractive sensor (1), and the diffractive image is generated by reflection from the diffractive sensor (1).

17. System according to anyone of claims from 14 to 16, further comprising a vision system (300) capable of detecting the diffraction image projected on the screen (200).

18. System according to the preceding claim, further comprising a control unit (400) operatively connected to the vision system (300) and configured to compare the diffraction image projected on the screen (200) to a reference diffraction image, and to determine the presence of the target analyte on the receptor layer (5) of the diffractive sensor (1) if the detected diffraction image is different from the reference diffraction image.

19. Method of detecting a target analyte in a sample, comprising the steps of:

    - providing a diffractive sensor (1) according to anyone of claims from 1 to 13;
    - applying the sample to the receptor layer (5) of said diffractive sensor (1);
    - hitting the diffractive sensor (1) with a laser light beam having a wavelength comprised in the visible spectrum, so that the diffractive sensor (1) generates a diffraction image visible to a naked eye;
    - comparing the diffraction image generated by the diffractive sensor (1) to a reference diffraction image obtained by hitting the diffractive sensor (1) with an equal laser light beam without the target analyte on the receptor layer (5);
    - determining the presence of the target analyte in the sample if the diffraction image generated by the diffractive sensor (1) is different from the reference diffraction image.

20. Method according to the preceding claim, comprising, after the step of applying the sample on the receptor layer (5) and before the step of hitting the diffractive sensor (1) with a laser light beam, a step of washing the diffractive sensor (1) for removing polluting substances or molecules or agents different from the target analyte from the receptor layer (5).

21. Method according to claim 19 or 20, wherein the receptor layer (5) of the diffractive sensor (1) is capable to be selectively bonded to a plurality of target analytes, the method further comprising the steps of:

- comparing the diffraction image produced by the diffractive sensor (1) to a plurality of stored diffraction images, each corresponding to a specific target analyte of the plurality of target analytes;
- determining the presence of a specific target analyte of the plurality of target analytes if the diffraction image generated by the diffractive sensor coincides with the stored diffraction image for that specific target analyte.

FIG. 1

FIG. 2

40 →

3

**FIG. 3**

30

40

**FIG. 4**

FIG. 5

FIG. 6

a)                                    b)

FIG. 7

a)                b)                c)                d)

FIG. 8

FIG. 9

Europäisches
Patentamt
European
Patent Office
Office européen
des brevets

# EUROPEAN SEARCH REPORT

**Application Number**

EP 24 16 4097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XUEFENG WANG ET AL: "Molecular layer detection on a diffractive optical balance", OPTICS LETTERS, OPTICAL SOCIETY OF AMERICA, US, vol. 37, no. 19, 1 October 2012 (2012-10-01), pages 4098-4100, XP001578722, ISSN: 0146-9592, DOI: 10.1364/OL.37.004098 [retrieved on 2012-09-26] | 1,3-8, 14,22,24 | INV. G01N21/47 |
| Y | * abstract * * page 4098, right-hand column, paragraph 1 * * page 4099, left-hand column, last paragraph - page 4100, right-hand column * * figures 1(a),3(b) * | 2,11,12, 17-21,23 | |
| X | US 2012/034291 A1 (AMSDEN JASON J [US] ET AL) 9 February 2012 (2012-02-09) * paragraphs [0048], [0064], [0067], [0092], [0093], [0102] * * figures 3A,10A * | 1-8,15, 16 | |
| | | | **TECHNICAL FIELDS SEARCHED (IPC)** |
| | | | G01N |
| X | US 2008/240543 A1 (BUDACH WOLFGANG ERNST GUSTAV [DE] ET AL) 2 October 2008 (2008-10-02) | 1,3-15 | |
| Y | * paragraphs [0033], [0034], [0039], [0080], [0086], [0117], [0125], [0129], [0130], [0136], [0220] * | 23 | |
| Y | US 4 647 544 A (NICOLI DAVID F [US] ET AL) 3 March 1987 (1987-03-03) * column 22, line 11 - line 43 * * figures 1,2c,4C * * column 15, line 15 - line 24 * * column 20, line 6 - line 23 * | 2,11,12, 17-21 | |

-/--

The present search report has been drawn up for all claims

| Place of search | Date of completion of the search | Examiner |
|---|---|---|
| Munich | 22 August 2024 | D'Alessandro, Davide |

Europäisches
Patentamt

European
Patent Office

Office européen
des brevets

# EUROPEAN SEARCH REPORT

Application Number

EP 24 16 4097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MARTINS EMILIANO R. ET AL: "Deterministic quasi-random nanostructures for photon control", NATURE COMMUNICATIONS, vol. 4, no. 1 24 October 2013 (2013-10-24), XP093122678, UK ISSN: 2041-1723, DOI: 10.1038/ncomms3665 Retrieved from the Internet: URL:https://www.nature.com/articles/ncomms 3665 * abstract * * figures 1,2 * | 2 | |
| Y | Jolly Xavier ET AL: "Deterministic aperiodic composite lattice-structured silicon thin films for photon management", arXiv.org, 19 May 2016 (2016-05-19), XP093122692, Ithaca DOI: 10.48550/arxiv.1605.06107 Retrieved from the Internet: URL:https://arxiv.org/ftp/arxiv/papers/160 5/1605.06107.pdf [retrieved on 2024-01-23] * page 1 - page 3 * * figures 1,2 * | 2 | TECHNICAL FIELDS SEARCHED (IPC) |

The present search report has been drawn up for all claims

| Place of search | Date of completion of the search | Examiner |
|---|---|---|
| Munich | 22 August 2024 | D'Alessandro, Davide |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 4097

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2024

| Patent document cited in search report | Publication date | Patent family member(s) | | Publication date |
|---|---|---|---|---|
| US 2012034291 A1 | 09-02-2012 | AU | 2010242010 A1 | 29-09-2011 |
| | | CA | 2789009 A1 | 04-11-2010 |
| | | EP | 2396276 A2 | 21-12-2011 |
| | | JP | 5717653 B2 | 13-05-2015 |
| | | JP | 2012517370 A | 02-08-2012 |
| | | US | 2012034291 A1 | 09-02-2012 |
| | | US | 2015272903 A1 | 01-10-2015 |
| | | WO | 2010126640 A2 | 04-11-2010 |
| US 2008240543 A1 | 02-10-2008 | AU | 2008233214 A1 | 09-10-2008 |
| | | CA | 2680064 A1 | 09-10-2008 |
| | | EP | 2137529 A1 | 30-12-2009 |
| | | JP | 2010523973 A | 15-07-2010 |
| | | KR | 20090128528 A | 15-12-2009 |
| | | NZ | 578655 A | 25-11-2011 |
| | | US | 2008240543 A1 | 02-10-2008 |
| | | WO | 2008121250 A1 | 09-10-2008 |
| US 4647544 A | 03-03-1987 | EP | 0167335 A2 | 08-01-1986 |
| | | US | 4647544 A | 03-03-1987 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3907507 A1

**Non-patent literature cited in the description**

- **RAFAEL ANTONIO SALINAS DOMINGUEZ** ; **MIGUEL ÁNGEL DOMINGUEZ JIMÉNEZ** ; **ABDÚ ORDUÑA DIAZ**. Antibody Immobilization in Zinc Oxide Thin Films as an Easy-Handle Strategy for Escherichia coli Detection. *ACS Omega*, 2020, vol. 5, 20473-20480
- **ANKE K. TRILLING** ; **JULES BEEKWILDERA** ; **HAN ZUILHOF**. Antibody orientation on biosensor surfaces: a minireview. *Analyst*, 2013, vol. 138, 1619
- **LU ZHANG** ; **YACINE MAZOUZI** ; **MICH&LE SALMAIN** ; **BO LIEDBERG** ; **SOUHIR BOUJDAY**. Antibody-Gold Nanoparticle Bioconjugates for Biosensors: Synthesis, Characterization and Selected Applications. *Biosensors and Bioelectronics*, 2020, vol. 165, 112370
- **ASTA MAKARAVICIUTE** ; **CAROLYN D. JACKSON** ; **PAUL A.MILLNER** ; **ALMIRA RAMANAVICIENE**. Considerations in producing preferentially reduced half-antibody fragments. *Journal of Immunological Methods*, 2016, vol. 429, 50-56
- **MONALISA PAL** ; **SANGHEE LEE** ; **DONGHOON KWON** ; **JEONGIN HWANG** ; **HYEONJEONG LEE** ; **SEOKYUNG HWANG** ; **SANGMIN JEON**. Direct immobilization of antibodies on Zn-doped Fe3O4 nanoclusters for detection of pathogenic bacteria. *Analytica Chimica Acta*, 2017, vol. 952, 81-87
- **MD. MORSALINE BILLAH** ; **CHRISTOPHER S. HODGES** ; **HENRY C.W. HAYS** ; **P.A. MILLNER**. Directed immobilization of reduced antibody fragments onto a novel SAM on gold for myoglobin impedance immunosensing.. *Bioelectrochemistry*, 2010, vol. 80, 49-54
- **HONGCHENG LIU** ; **KIMBERLY MAY**. Disulfide bond structures of IgG molecules. *mAbs*, 2012, vol. 4 (1), 17-23

- **ROBERTO REVERBERI** ; **LORENZO REVERBERI**. Factors affecting the antigen-antibody reaction. *Blood Transfus*, 2007, vol. 5, 227-240
- **HARSH SHARMA** ; **RAJ MUTHARASAN**. Half Antibody Fragments Improve Biosensor Sensitivity without Loss of Selectivity. *Anal. Chem.*, 2013, vol. 85, 2472-2477
- **WOOCHANG LEE** ; **BYUNG-KEUN OH** ; **WON HONG LEE** ; **JEONG-WOO CHOI**. Immobilization of antibody fragment for immunosensor application based on surface plasmon resonance.. *Colloids and Surfaces B: Biointerfaces*, 2005, vol. 40, 143-148
- **YUANLI SONG** ; **HUI CAI** ; **ZHIJUN TAN** ; **NESREDIN MUSSA** ; **ZHENG-JIAN LI**. Mechanistic insights into inter-chain disulfide bond reduction of IgG1 and IgG4 antibodies. *Applied Microbiology and Biotechnology*, 2022, vol. 106, 1057-1066
- **NICHOLAS G. WELCH** ; **JUDITH A. SCOBLE** ; **BENJAMIN W. MUIR et al.** Orientation and characterization of immobilized antibodies for improved immunoassays. *Biointerphases*, 2017, vol. 12, 02D301
- **ARKADY A. KARYAKIN** ; **GALINA V. PRESNOVA** ; **MAYA YU. RUBTSOVA** ; **ALEXEY M. EGOROV**. Oriented Immobilization of Antibodies onto the Gold Surfaces via Their Native Thiol Groups. *Anal. Chem.*, 2000, vol. 72, 3805-3811
- **NUR MUSTAFAOGLU** ; **TANYEL KIZILTEPE** ; **BASAR BILGICER**. Site-Specific Conjugation of Antibody on Gold Nanoparticle Surface for One-Step Diagnosis of Prostate Specific Antigen with Dynamic Light Scattering. *Nanoscale.*, 29 June 2017, vol. 9 (25), 8684-8694
- **HARRY W SCHROEDER JR** ; **LISA CAVACINI**. Structure and Function of Immunoglobulins. *Allergy Clin Immunol.*, February 2010, vol. 125 (2 0 2), S41-S52